# EUROPÄISCHE PATENTSCHRIFT

(11) **EP 1 175 379 B1**
(45) Veröffentlichungstag und Bekanntmachung des Hinweises auf die Patenterteilung: **02.01.2004**
(21) Anmeldenummer: 00926979.6
(22) Anmeldetag: 18.04.2000
(51) Int. Cl.: C07B 53/00, C07C 29/50, C07C 31/20, C07C 31/42, C07C 33/26, C07C 35/14, C07C 41/26, C07C 43/253, C07C 319/20, C07C 323/12, C07D 319/06, C07F 7/08

(54) **VERFAHREN ZUR ASYMMETRISCHEN DIHYDROXYLIERUNG VON OLEFINEN MITTELS OSMIUM-KATALYSATOREN**
METHOD FOR THE ASYMMETRIC DIHYDROXYLATION OF OLEFINS, USING OSMIUM CATALYSTS
PROCEDE DE DIHYDROXYLATION ASYMETRIQUE D'OLEFINES A L'AIDE DE CATALYSEURS A BASE D'OSMIUM

(30) Priorität: 25.04.1999 DE 19920039
(43) Veröffentlichungstag der Anmeldung: 30.01.2002
(73) Patentinhaber: Bayer Chemicals AG, 51368 Leverkusen (DE)
(72) Erfinder: BELLER, Matthias, D-18119 Rostock (DE); DÖBLER, Christian, D-18107 Lichtenhagen-Dorf (DE); MEHLTRETTER, Gerald, D-18057 Rostock (DE); SUNDERMEIER, Uta, D-18055 Rostock (DE)
(86) Internationale Anmeldenummer: PCT/EP2000/003494
(87) Internationale Veröffentlichungsnummer: WO 2000/064844

(56) Entgegenhaltungen:
- WO-A-89/06225
- US-A- 5 260 461
- K.B. SHARPLESS, ET AL.: "The osmium-catalysed asymmetric dihydroxylation: a new ligand class and process improvement" JOURNAL OF ORGANIC CHEMISTRY, Bd. 57, Nr. 10, 8. Mai 1992 (1992-05-08), Seiten 2768-2771, XP002144279 American Chemical Society, Washington, DC, US ISSN: 0022-3263

## Beschreibung

Die vorliegende Erfindung betrifft ein Verfahren zur Herstellung von chiralen 1,2-Diolen aus Olefinen mit Katalysatoren auf Basis von Osmium Verbindungen.

Chirale 1,2-Diole haben technische Bedeutung als Feinchemikalien und als Zwischenprodukte für Arzneimittel sowie Agrowirkstoffe.

Die Standardmethode zur Synthese von chiralen 1,2-Diolen ist die sogenannte Sharpless-Dihydroxylierungsreaktion, bei der Olefine in Gegenwart von Osmiumtetroxid, chiralen Stickstoffliganden und überstöchiometrischen Mengen von Kaliumhexacyanoferrat und Kaliumcarbonat als Oxidationsmittel umgesetzt werden. Übersichtsartikel, die diese Methodik beschreiben, findet man beispielsweise in *"Asymmetric Dihydroxylation Reactions"* M. Beller, K.B. Sharpless, in B. Comils, W. A. Herrmann (Eds.) VCH, **1996,** Weinheim und H.C. Kolb, M.S. Van Nieuwenhze, K.B. Sharpless, Chem. Rev. **1994,** 94, 2483

Ein entscheidender Nachteil der Sharpless-Dihydroxylierung ist der Einsatz von mehreren Äquivalenten Kaliumhexacyanoferrat als Oxidationsmittel (Y. Ogino, H. Chen, H.L. Kwong, K.B. Sharpless, *Tetrahedron Lett.* **1991,** 32, 3965 US 5 260 461). Neben dem Preis des Oxidationsmittels sind insbesondere der große Anfall von Salz- und Metallabfällen ökologisch unvorteilhaft. Damit ist für eine Synthese der Diole in technisch größerem Maßstab sowohl der Preis als auch die überstöchiometrische Menge an aufzuwendendem Eisenkomplex (3 Mol = 990 g für 1 Mol Substrat) unter Zusatz von Kaliumcarbonat (3 Mol = 420 g) ein erheblicher Nachteil. Auch bei Verfahren zur elektrochemischen Oxidation des bei der Reaktion entstehenden Na₄[Fe(CN)₆] zu Na₃[Fe(CN)₆] (Sepracor Inc. (Y. Gao, C.M. Zepp), PCT Int. Appl. WO 9.317.150, 1994; Anon., *Chem. Eng. Mews*, **1994,** *72 (24)*, 41) ist eine großtechnische Umsetzung schwierig, da elektrochemische Verfahren aufgrund der benötigten apparativen Voraussetzungen generell zu teuer sind.

Obwohl in der Literatur kostengünstigere Oxidationsmittel für Dihydroxylierungen bekannt sind (bspw. Chlorate; K.A. Hofmann, Chem. **1912,** 45, 3329; H₂O₂ in tert. Butanol: N.A. Milas, J.-H. Trepagnier, J.T. Nolan, M.Ji. Iliopolus, J. *Am. Chem. Soc.* **1959,** 81, 4730: tert.-Butylhydroperoxid in Gegenwart von Et₄NOH; K.B. Sharpless, K. Akashi, J. *Am. Chem. Soc*. **1976,** 98, 1986: P.H.J. Carlsen, T. Katsuki, V.S. Martin, K.B. Sharpless, *J. Org. Chem.* **1981,** 46, 3936; F.X. Webster, J. Rivas-Enterrios, R.M. Silverstein, *J. Org. Chem.* **1987,** 52, 689; V.S. Martin, M.T. Nunez, C.E. Tonn, *Tetrahedron Lett.* **1988,** 29, 2701; M. Caron, P.R. Carlier, K.B. Sharpless, *J. Org. Chem.* **1988,** 53, 5185, tertiäre Aminoxide und in den meisten Fällen N-Methylmorpholin-N-oxid; W. P. Schneider, A.V. Mcintosh, US 2.769.824 (1956); V. Van Rheenen, R.C. Kelly, D.Y. Cha, *Tetrahedron Lett.* **1976,** 17, 1973) erlauben alle genannten Prozesse keine Darstellung von chiralen Diolen mit guten Enantioselektivitäten.

Zur Vermeidung der aufgezeigten Nachteile des bekannten katalytischen Verfahrens mit Kaliumhexacyanoferrat ist es Aufgabe der Erfindung, ein neues Verfahren zur asymmetrischen Dihydroxylierung zu entwickeln, das chirale 1,2-Diole in hoher Ausbeute, Enantioselektivität und Reinheit liefert, wobei ein kostengünstiges Reoxidationsmittel eingesetzt wird, und das für eine großtechnische Durchführung geeignet ist.

Diese Aufgabe wird gelöst durch ein Verfahren zur asymmetrischen Dihydroxylierung von Olefinen mittels Osmium-Katalysatoren, in dem erfindungsgemäß mono-, bi- und/oder polyfunktionelle 1,2-Diole der Formel (I)

R¹R²C(OH)-C(OH)R³R⁴ (I)

worin
- R¹ bis R⁴: unabhängig voneinander Wasserstoff, Alkyl, CN, COOH, COO-Alkyl, COO-Aryl, CO-Alkyl, CO-Aryl, O-Alkyl, O-Aryl, O-CO-Aryl, O-CO-Alkyl, OCOO-Alkyl, N-Alkyl₂, NH-Alkyl, N-Aryl₂, NH-Aryl, NO, NO₂, NOH, Aryl, Fluor, Chlor, Brom, Iod, NO₂, SiAlkyl₃, CHO, SO₃H, SO₃-Alkyl, SO₂-Alkyl, SO-Alkyl, CF₃, NHCO-Alkyl, CONH₂, CONH-Alkyl, NHCOH, NHCOO-Alkyl, CHCHCO₂-Alkyl, CHCHCO₂H, PO-(Aryl)₂, PO-(Alkyl)₂, PO₃H₂, PO(O-Alkyl)₂ bedeuten, und wobei Alkyl für eine aliphatische Kohlenstoffgruppe mit 1 bis 18 C-Atomen, die linear, verzweigt und/oder auch cyclisch ist, steht und Aryl einen 4 bis zu 14 C-Atome enthaltenden fünf-, sechs- oder siebengliedrigen aromatischen Ring, wobei dieser Ring anelliert sein kann und 0 bis 3 Heteroatome wie N, O, S enthalten kann, bedeutet und wobei die Alkyl- als auch die Arylgruppe gegebenenfalls bis zu sechs weitere Substituenten tragen können, die unabhängig voneinander Wasserstoff, Alkyl, O-Alkyl, OCO-Alkyl, O-Aryl, Aryl, Fluor, Chlor, Brom, Iod, OH, NO₂, NO, SiAlkyl₃, CN, COOH, CHO, SO₃H, NH₂, NH-Alkyl, N-Alkyl₂, PO-Alkyl₂, SO₂-Alkyl, SO-Alkyl, CF₃, NHCO-Alkyl, COO-Alkyl, CONH₂, CO-Alkyl, NHCOH, NHCOO-Alkyl, CO-Aryl, COO-Aryl, PO-Aryl₂, PO₃H₂, PO(O-Alkyl)₂, SO₃-Alkyl bedeuten, wobei Alkyl und Aryl die oben genannte Bedeutung haben,
durch Umsetzung von Olefinen der allgemeinen Formel (II)

R¹R²C=CR³R⁴ (II)

worin
R¹ bis R⁴ die oben genannten Bedeutungen besitzen,
mit molekularem Sauerstoff in Gegenwart einer katalytischen Menge einer Osmiumverbindung und eines chiralen Amins in Wasser oder einem Wasser enthaltenden Lösemittelgemisch bei einem pH-Wert von 7,5 bis 13 erhalten werden.

Insbesondere werden zur Herstellung von Verbindungen der Formel (I) Olefine der Formel (II) eingesetzt, wobei die Substituenten R¹ bis R⁴ unabhängig voneinander Wasserstoff, Alkyl, CN, COOH, COO-Alkyl, COO-Aryl, CO-Alkyl, CO-Aryl, O-Alkyl, O-Aryl, N-Alkyl₂, Aryl, Fluor, Chlor, Brom, Iod, CHO, CF₃, NHCO-Alkyl, CONH₂, CONH-Alkyl, NHCOO-Alkyl bedeuten. Dabei haben Alkyl und Aryl die oben genannten Bedeutungen.

Besonders bevorzugt ist ein Verfahren, bei dem Diole der Formel (I) hergestellt werden, worin R¹ bis R⁴ unabhängig voneinander Wasserstoff, Alkyl, CN, COOH, COO-Alkyl, CO-Alkyl, CO-Aryl, O-Alkyl, O-Aryl, Aryl, Fluor, Chlor, Brom, CHO, NHCO-Alkyl bedeuten. Dabei haben Alkyl und Aryl die oben genannten Bedeutungen.

Das erfindungsgemäße Verfahren wird in Gegenwart von Wasser durchgeführt. Als vorteilhaft hat es sich erwiesen, neben dem Olefin ein weiteres organisches Lösemittel zuzusetzen. Das erfindungsgemäße Verfahren kann bei verschiedenen Olefinen auch im Gemisch Olefin/Wasser ohne weiteres Lösemittel durchgeführt werden. Als weitere Lösungsmittel finden im allgemeinen inerte organische Lösungsmittel Verwendung. Geeignet sind aliphatische Ether, aromatische oder aliphatische Kohlenwasserstoffe, Alkohole und Ester, halogenierte Kohlenwasserstoffe, dipolar aprotische Lösungsmittel wie Dialkylsulfoxide, N,N-Dialkylamide von aliphatischen Carbonsäuren sowie deren Gemische. Hierbei sind Alkohole, Ester und Ether bevorzugt. Als Wasserphase wird im allgemeinen eine basische wässrige Lösung mit einem pH-Wert von 7,5 bis 13 verwendet. Der basische pH-Wert der Lösung wird durch den Zusatz einer Base zum Wasser erzielt. Generell ist es vorteilhaft in gepufferten wässrigen Lösungen, vorzugsweise bei pH 8 bis 13, zu arbeiten. Die gepufferte Lösung wird durch den Zusatz von bekannten Puffern zu Wasser zubereitet.

Mitunter ist es für die Abtrennung der Diolprodukte vorteilhaft, wenn an Stelle von Wasser oder gepufferten wässrigen Lösungen als Lösungsmittel eine wässrige Salzlösung oder gepufferte wässrige Salzlösung - beispielsweise wässrige Lösung eines Alkali- oder Erdalkalihalogenids - eingesetzt wird.

Als Oxidationsmittel wird im erfindungsgemäßen Verfahren molekularer Sauerstoff oder eine Gasmischung, die molekularen Sauerstoff enthält, eingesetzt. Bevorzugt sind Gasmischungen, die mindestens 15 Volumenprozent Sauerstoff enthalten. Besonders bevorzugt sind Luft und Sauerstoffgas mit einem Sauerstoffanteil von >95 %.

Die Reaktion läuft vorzugsweise bei Temperaturen von 20 bis 150°C ab. In vielen Fällen hat es sich bewährt, bei Temperaturen von 30 bis 120°C, bevorzugt 40 bis 80°C, zu arbeiten. Das erfindungsgemäße Verfahren kann drucklos, z.B. durch Durchleiten von Sauerstoff durch die Reaktionslösung, durchgeführt werden. Jedoch ist es für die Reaktionsgeschwindigkeit vorteilhaft, wenn ein Sauerstoffüberdruck angewandt wird. Das Verfahren kann bei Drücken bis zu 200 bar umgesetzt werden, wobei üblicherweise nur bis zu einem Druck von 60 bar und vorzugsweise im Bereich des Normaldrucks bis zu 20 bar gearbeitet wird.

Als chirale Liganden werden in der Literatur bekannte (H.C. Kolb, M.S. Van Nieuwenhze', and K.B. Sharpless, *Chem Rev*. **1994,** 94, 2483-2547) chirale Amine wie Diaminocyclohexanderivate, substituierte Diaminoethane, Bis-piperazin, Bispyrrolidin-, Bis-tetrahydropyridin-Verbindungen, 1,4-Diazabicyclo[2,2,2]octan-Derivate, substituierte Isooxazolidine, insbesondere (DHQD)₂PHAL (Hydrochinidin-1,4-phthalazindiyl-diether) und (DHQ)₂PHAL (Hydrochinin-1,4-phthalazindiyl-diether) sowie (DHQ)₂Pyr [Hydrochinin-(2,5-diphenyl-4,6-pyrimidinyl)-diether] eingesetzt.

Die eingesetzten Osmiumkatalysatoren sind in der Regel Osmiumverbindungen in den Oxidationsstufen +8 und +6. Es ist jedoch auch möglich, Osmiumpräkatalysatoren in niedrigen Oxidationsstufen einzusetzen. Diese werden unter den Reaktionsbedingungen in die katalytisch aktiven Os(VIII)- und Os(VI)-Spezies umgewandelt. Als Osmium-Katalysatoren oder Katalysatorvorstufen können beispielsweise OsO₄, K₂Os₂(OH)₄, Na₂Os₂(OH)₄, Os₃(CO)₁₂, OsCl₃, H₂OsCl₆, [CF₃SO₃Os(NH₃)₅](O₃SCF₃)₂, OsO₄ auf Vinylpyridin, Bu^{t}NOsO₃ eingesetzt werden.

Nach dem erfindungsgemäßen Verfahren wird der Osmiumkatalysator in katalytischen Mengen bezüglich des Olefins eingesetzt. Generell werden zwischen 0,2 und 0,00001 Äquivalente, bezogen auf Olefin, bevorzugt 0,1 bis 0,0001 und besonders bevorzugt 0,08 bis 0,0005 Äquivalente verwendet.

Das Verhältnis Amin zu Osmium beträgt zwischen 0,01: 1 bis 1000 : 1, vorzugsweise zwischen 0,1 : 1 bis 100 : 1. Besonders bevorzugt werden Verhältnisse von Amin zu Osmium von 1 : 50 bis 50 : 1 verwendet.

Bei Einsatz von sterisch anspruchsvollen Olefinen, besonders tri- und tetrasubstituierten Olefinen ist es mitunter vorteilhaft, einen Co-Katalysator zur Hydrolyse der intermediär entstehenden Osmatester zuzusetzen. Dieser Co-Katalysator ist ein die Hydrolyse vereinfachendes Amid wie beispielsweise Sulfonamide und/oder Carbonsäureamide. Besonders bevorzugt ist der Zusatz von Methylsulfonsäureamid.

Der Co-Katalysator wird in einer Menge von 0,01 Mol-% bis 10 Mol-% (bezogen auf Olefin) und bevorzugt von 0,1 bis 5 Mol-% eingesetzt.

Der besondere Vorteil des erfindungsgemäßen Verfahrens ist die Verwendung von Sauerstoff oder sauerstoffenthaltenden Gasen als Reoxidationsmittel. Trotz des vergleichsweise schwierigen Reoxidationsprozesses können hohe Enantioselektivitäten erreicht werden. Die Katalysatorproduktivität kann erhöht werden, in dem die einmal eingesetzte wässrige Katalysatorphase erneut mit Olefin versetzt wird. Dadurch werden die Katalysatorkosten für das erfindungsgemäße Verfahren minimiert, so dass sogar technische Prozesse ökonomisch durchführbar sind.

Das erfindungsgemäße Verfahren ist insofern besonders überraschend und neu, da in der Vergangenheit keine asymmetrischen osmiumkatalysierten Dihydroxylierungsreaktionen zu 1,2-Diolen mit Sauerstoff als Reoxidanz bekannt sind. Die im erfindungsgemäßen Verfahren beschriebene neue Kombination von Ligandenzusatz, der die Dihydroxylierung beschleunigt und die Durchführung des Verfahrens in einer gepufferten stark basischen Lösung führt überraschenderweise zu einem enantioselektiven Dihydroxylierungsprozess auch in Gegenwart von Sauerstoff. Das erfindungsgemäße Verfahren zeigt hier erstmals, dass die Vorstellungen in der bekannten Literatur zur osmiumkatalysierten Dihydroxylierung mit Sauerstoff falsch sind.

Die besonderen Vorteile des neuen Prozesses bestehen in dem Preisvorteil des Oxidationsmittels, der Einfachheit der Durchführung und der großen Selektivität des Verfahrens im Vergleich zum bekannten Verfahren mit Kaliumhexacyanoferrat.

Die erfindungsgemäß hergestellten chiralen 1,2-Diole können unter anderem eingesetzt werden als Vorprodukte für Agrochemikalien, Kosmetika, Pharmazeutika und chiralen Polymeren.

### Ausführungsbeispiele

### Beispiel 1

In ein Schlenkgefäß werden 18,4 mg K₂OsO₄ x 2H₂O (0,05 mmol) eingewogen. Dazu werden unter Rühren mittels eines Magnetrührers 25 ml 0,4 - 0,5 molarer Pufferlösung Na₃PO₄/Na₂HPO₄ vom pH 11,2 und 10 ml 2-Methyl-2-propanol gegeben, es bilden sich 2 Phasen. Das Gefäß wird in einem Wasserbad auf 50°C erwärmt und mit Sauerstoff gespült. Nach Zugabe von 173 µl Styren (1,5 mmol) wird das Reaktionsgefäß mit einer Bürette, gefüllt mit Sauerstoff, verbunden, und die Reaktionslösung wird bei 50°C unter leichtem O₂-Überdruck (ca. 50 cm Wassersäule) 24 Stunden gerührt.

Es wird wie im folgenden beschrieben aufgearbeitet:
Zu der Reaktionslösung werden 2 g Natriumbisulfit und 10 ml Essigester gegeben. Nach 10-minütigem Rühren wird die obere organische Phase abgetrennt und die wässrige Phase mit 10 ml Essigester ausgeschüttelt. Die organischen Phasen werden vereinigt, mit wasserfreiem Natriumsulfat getrocknet, im Rotationsverdampfer wird bis zur Trockne eingeengt.
Man erhält 130 mg (R)/(S)-1-Phenyl-1,2-ethandiol, 63 %.

Zur Isolierung eventuell entstandener saurer Produkte wird die wässrige Lösung angesäuert und 2 x mit 15 ml Ether ausgeschüttelt. Man erhält 20 mg eines kristallinen Produktes, das zu mehr als 90 % aus Benzoesäure besteht.

### Beispiel 2

Es wird wie im Beispiel 1 beschrieben verfahren, zu dem Osmiumsalz werden 7,8 mg (0,01 mmol) (DHQD)₂PHAL (Hydrochinidin-1,4-phthalazindiyi-diether) hinzugefügt. Man erhält 155 mg (R)-(+)-1-Phenyl-1,2-ethandiol (75 %), ee 65 % (HPLC) und 30 mg Benzoesäure.

### Beispiel 3

In ein Schlenkgefäß werden 18,4 mg K₂OsO₄ x 2H₂O (0,05 mmol) und 7,8 mg (0,01 mmol) (DHQD)₂PHAL eingewogen. Dazu werden unter Rühren mittels eines Magnetrührers 25 ml 0,3 molarer Pufferlösung Borax/NaOH vom pH 10,2, 4 g NaCl und 10 ml 2-Methyl-2-propanol gegeben, es bilden sich 2 Phasen. Das Gefäß wird in einem Wasserbad auf 50°C erwärmt und mit Sauerstoff gespült. Nach Zugabe von 288 µl Styren (2,5 mmol) wird das Reaktionsgefäß mit einer Bürette, gefüllt mit Sauerstoff, verbunden, und die Reaktionslösung wird bei 50°C unter leichtem O₂-Überdruck (ca. 50 cm Wassersäule) 24 Stunden gerührt. Es wird wie Beispiel 1 angegeben aufgearbeitet.
Man erhält 200 mg (R)-(+)-1-Phenyl-1,2-ethandiol (58 %), ee 82 % (HPLC) und 40 mg Benzoesäure.

### Beispiel 4

Wie in Beispiel 1 beschrieben werden 1,5 mmol Styren mit 18,4 mg K₂OsO₄ x 2H₂O (0,05 mmol) und 7,8 mg (0,01 mmol) (DHQD)₂PHAL umgesetzt, die Reaktionstemperatur betrug hier 30°C, die Reaktionszeit 62 Stunden.
Man erhält nach Aufarbeitung 107 mg überwiegend (R)-(+)-1-Phenyl-1,2-ethandiol (52 %), ee 71 % (HPLC) und 40 mg Benzoesäure.

### Beispiel 5

Es werden wie im Beispiel 1 beschrieben 18,4 mg K₂OsO₄ x 2H₂O (0,05 mmol) mit 1,5 mmol Styren umgesetzt. Vor der Styrenzugabe werden 8,8 mg (0,01 mmol) (DHQ)₂Pyr [Hydrochinin-(2,5-diphenyl-4,6-pyrimidinyl)-diether] hinzugefügt.
Man erhält 141 mg überwiegend (S)-(-)-1-Phenyl-1,2-ethandiol (68 %), ee 23 % (HPLC) und 40 mg Benzoesäure.

### Beispiel 6

Es wird wie im Beispiel 1 angegeben verfahren. Als Substrat werden 231 mg 2-Vinylnaphthalin (1.5 mmol) mit 18,4 mg K₂OsO₄ x 2H₂O (0,05 mmol) unter Zusatz von 7,8 mg (0,01 mmol) (DHQ)₂PHAL [Hydrochinin-1,4-phthalazindiyl-diether) umgesetzt, die Reaktionszeit betrug abweichend hier 7 Stunden. Man erhält nach Aufarbeitung 227 mg (S)-1-(2-Naphthyl)-1,2-ethandiol (80 %), ee 82 % (HPLC). Aus der etherischen Lösung werden 34 mg eines kristallinen Produktes erhalten, welches überwiegend aus 2-Naphthalcarbonsäure besteht.

### Beispiel 7

Analog Beispiel 1 werden 18,4 mg K₂OsO₄ x 2H₂O (0,05 mmol) mit 195 µl (1,5 mmol) α-Methylstyren unter Zusatz von 7,8 mg (0,01 mmol) (DHQ)₂PHAL in dem angegebenen 2-Phasensystem umgesetzt.
Man erhält nach Aufarbeitung in der angeführten Weise 180 mg überwiegend (S)-2-Phenyl-1,2-propandiol (79 %), ee 60 % (GC).

### Beispiel 8

Analog Beispiel 1 werden 18,4 mg K₂OsO₄ x 2H₂O (0,05 mmol) mit 130 µl (1 mmol) trans-β-Methylstyren unter Zusatz von 7,8 mg (0,01 mmol) (DHQD)₂PHAL umgesetzt.
Man erhält nach der üblichen Aufarbeitung 126 mg (R,R)-1-Phenyl-1,2-propandiol (80 %).

### Beispiel 9

In ein Schlenkgefäß werden 7,4 mg K₂OsO₄ x 2H₂O (0,02 mmol) eingewogen. Dazu werden unter Rühren mittels eines Magnetrührers 25 ml einer Pufferlösung vom pH 10,4 dargestellt aus 0,5 molarer K₂HPO₄-Lösung und 2 molarer NaOH, sowie 10 ml 2-Methyl-2-propanol gegeben, es bilden sich 2 Phasen. Das Gefäß wird in einem Wasserbad auf 50°C erwärmt und mit Sauerstoff gespült. Nach Zugabe von 230 µl Styren (2 mmol) wird das Reaktionsgefäß mit einer Bürette, gefüllt mit Sauerstoff, verbunden, und die Reaktionslösung wird bei 50°C unter leichtem O₂-Überdruck (ca. 50 cm Wassersäule) 24 Stunden gerührt.

Es wird wie im Folgenden beschrieben aufgearbeitet:
Zu der Reaktionslösung werden 2 g Natriumbisulfit und 20 ml Essigester gegeben. Nach 10-minütigem Rühren wird die obere organische Phase abgetrennt. Mittels GC werden Dialkohol sowie nicht umgesetztes Olefin bestimmt.
Ausbeute an 1-Phenyl-1,2-ethandiol: 43 % (Selektivität 57 %).

### Beispiel 10

Es wird wie in Beispiel 9 angeführt verfahren, jedoch werden zu dem Osmiumsalz 0,02 mmol (DHQD)₂PHAL hinzugefügt.

Ausbeute an (R)-1-Phenyl-1,2-ethandiol: 49 % (Selektivität 74 %), ee 89 % (HPLC).

### Beispiel 11

Es wird wie in Beispiel 9 angeführt verfahren, jedoch werden 0,06 mmol (DHQD)₂PHAL hinzugefügt. Als Substrat werden 308 mg 2-Vinylnaphthalin (2 mmol) eingesetzt, Dialkohol sowie nicht umgesetztes Olefin werden hier mittels HPLC bestimmt.

Ausbeute an (R)-1-(2-Naphthyl)-1,2-ethandiol: 55 % (Selektivität 76 %), ee 93 % (HPLC).

### Beispiel 12

Wie in Beispiel 9 beschrieben, jedoch unter Einsatz einer Pufferlösung vom pH 11,2 werden 7,4 mg K₂OsO₄ x 2H₂O (0,02 mmol)/0,06 mmol (DHQD)₂PHAL mit 318 µl 1-Phenyl-1-cyclohexen (2 mmol) umgesetzt.

Ausbeute an (1R,2R)-1-Phenyl-1,2-cyclohexandiol: 80 % (Selektivität 83 %), ee 90 % (HPLC).

### Beispiel 13

Wie in Beispiel 9 beschrieben, jedoch in einer Reaktionszeit von 19 h, werden 3,7 mg K₂OsO₄ x 2H₂O (0,01 mmol)/0,03 mmol (DHQD)₂PHAL mit 260 µl α-Methylstyren (2 mmol) umgesetzt.

Ausbeute an (R)-2-Phenyl-1,2-propandiol: 96 % (Selektivität 96 %), ee 81 % (GC).

### Beispiel 14

Es wird wie in Beispiel 13 angeführt verfahren, als Ligand wird (DHQD)₂PYR (Hydrochinidin-[2,5-diphenyl-4,6-pyrimidindiyl]-diether) eingesetzt.

Ausbeute an (R)-2-Phenyl-1,2-propandiol: 95 % (Selektivität 95 %), ee 43 % (GC.

### Beispiel 15

Es wird wie in Beispiel 13 angeführt verfahren, als Ligand wird (DHQD)₂AQN (Hydrochinidin-[anthrachinon-1,4-diyl]-diether) eingesetzt.

Ausbeute an (R)-2-Phenyl-1,2-propandiol: 96 % (Selektivität 96 %), ee 65 % (GC).

### Beispiel 16

Es wird wie in Beispiel 13 angeführt verfahren, als Ligand werden 0,006 mmol (DHQD)₂PHEN (Hydrochinidin-9-phenanthrylether) eingesetzt.

Ausbeute an (R)-2-Phenyl-1,2-propandiol: 94 % (Selektivität 96 %), ee 42 % (GC).

### Beispiel 17

Analog Beispiel 13 wird 1-Octen umgesetzt, Reaktionszeit 15 h.

Ausbeute an (R)-1,2-Octandiol: 98 % (Selektivität 99 %), ee 65 % (HPLC, Bisbenzoat).

### Beispiel 18

Wie in Beispiel 9 angeführt, jedoch in einer Reaktionszeit von 12 h und unter Einsatz einer Pufferlösung vom pH 11,2 werden 7,4 mg K₂OsO₄ x 2H₂O (0,02 mmol)/0,06 mmol (DHQD)₂PHAL mit 240 µl 1-Methyl-1-cyclohexen (2 mmol) umgesetzt.

Ausbeute an (1R,2R)-1-Methyl-1,2-cyclohexandiol: 82 % (Selektivität 85 %), ee 49 % (HPLC, Bisbenzoat).

### Beispiel 19

Analog Beispiel 9, jedoch in einer Reaktionszeit von 6 h werden 320 µl Allyltrimethylsilan (2 mmol) umgesetzt.

Ausbeute an (S)-3-(Trimethylsilyl)-1,2-propandiol: 79 % (Selektivität 89 %), ee 15 % (HPLC, Bisbenzoat).

### Beispiel 20

Wie in Beispiel 9 beschrieben, jedoch in einer Reaktionszeit von 18 h und unter Einsatz einer Pufferlösung vom pH 12,0 werden 14,7 mg K₂OsO₄ x 2H₂O (0,04 mmol)/0,12 mmol (DHQD)₂PHAL mit 380 µl tmns-5-Decen (2 mmol) umgesetzt.

Ausbeute an (R,R)-5,6-Decandiol: 95 % (Selektivität 98 %), ee 88 % (HPLC, Bisbenzoat).

### Beispiel 21

Analog Beispiel 20 werden 245 µl 2-Methyl-2-penten (2 mmol) bei pH 11,2 umgesetzt.

Ausbeute an (2R,3R)-2-Methyl-2,3-pentandiol: 88 % (Selektivität 87 %), ee 87 % (HPLC, Bisbenzoat).

### Bcispiel 22

Analog Beispiel 20 werden 240 µl 2-Vinyl-1,3-dioxolan (2 mmol) bei pH 10,4 umgesetzt.

Ausbeute an (S)-2-(1,2-Dihydroxyethyl)-1,3-dioxolan: 63 % (Selektivität 86 %), ee 23 % (HPLC, Bisbenzoat).

### Beispiel 23

Analog Beispiel 22 werden 692 mg 1H,1H,2H-Perfluor-1-octen (2 mmol) mit (DHQD)₂AQN als Ligand umgesetzt.

Ausbeute an 1H,1H,2H-Perfluoroctan-1,2-diol: 40 % (Selektivität 83 %), ee 45 % (HPLC, Bisbenzoat).

### Beispiel 24

Wie in Beispiel 9 beschrieben werden 7,4 mg K₂OsO₄ x 2H₂O (0,02 mmol)/0,06 mmol (DHQD)₂PHAL mit 275 ml Allylphenylether (2 mmol) umgesetzt, Reaktionszeit 18 h.

Ausbeute an (S)-3-Phenoxy-1,2-propandiol: 80 % (Selektivität 95 %), ee 74 % (HPLC).

### Beispiel 25

Analog Beispiel 24 werden 295 µl Allylphenylsulfid (2 mmol) mit (DHQD)₂AQN als Ligand umgesetzt.

Ausbeute an (S)-(2,3-Dihydroxypropyl)-phenylsulfid: 67 % (Selektivität 92 %), ee 63 % (HPLC).

### Beispiel 26

In ein in einem Druckautoklaven befindliches Glasgefäß werden 0,002 mmol K₂OsO₄ x 2H₂O gelöst in Wasser, 0,030 mmol (DHQD)₂PHAL und 25 ml einer Pufferlösung vom pH 10,4, dargestellt aus 0,5 molarer K₂HPO₄-Lösung und 2 molarer NaOH sowie 12 ml 2-Methyl-2-propanol gegeben, unter Rühren mittels eines Magnetrührers bilden sich 2 Phasen. Nach Zugabe von 260 µl α-Methylstyren (2 mmol) wird ein Druck von 3 bar Sauerstoff aufgegeben und das Autoklavengefäß auf 50 bis 55°C erwärmt.

Nach 24 h wird wie in Beispiel 9 angeführt aufgearbeitet.

Ausbeute an (R)-2-Phenyl-1,2-propandiol: 93 % (Selektivität 93 %), ee 78 % (GC).

### Beispiel 27

Wie in Beispiel 26 beschrieben, werden 0,001 mmol K₂OsO₄ x 2H₂O/0,015 mmol (DHQD)₂PHAL mit 260 µl α-Methylstyren (2 mmol) bei 5 bar O₂-Druck umgesetzt.

Ausbeute an (R)-2-Phenyl-1,2-propandiol: 94 % (Selektivität 94 %), ee 77 % (GC).

### Beispiel 28

Es wird wie in Beispiel 26 angeführt verfahren, jedoch werden 8 bar Preßluft anstelle von reinem Sauerstoff auf den Autoklaven gegeben.

Ausbeute an (R)-2-Phenyl-1,2-propandiol: 80 % (Selektivität 93 %), ee 80 % (GC).

## Patentansprüche

1. Verfahren zur asymmetrischen Dihydroxylierung von Olefinen mittels Osmium-Katalysatoren zur Herstellung von mono-, bi- und/oder polyfunktionellen chiralen 1,2-Diolen der Formel (I)
R¹R²C(OH)-C(OH)R³R⁴ (I)
worin
R¹ bis R⁴ unabhängig voneinander Wasserstoff, Alkyl, CN, COOH, COO-Alkyl, COO-Aryl, CO-Alkyl, CO-Aryl, O-Alkyl, O-Aryl, O-CO-Aryl, O-CO-Alkyl, OCOO-Alkyl, N-Alkyl₂, NH-Alkyl, N-Aryl₂, NH-Aryl, NO, NO₂, NOH, Aryl, Fluor, Chlor, Brom, Iod, NO₂, SiAlkyl₃, CHO, SO₃H, SO₃-Alkyl, SO₂-Alkyl, SO-Alkyl, CF₃, NHCO-Alkyl, CONH₂, CONH-Alkyl, NHCOH, NHCOO-Alkyl, CHCHCO₂-Alkyl, CHCHCO₂H, PO-(Aryl)₂, PO-(Alkyl)₂, PO₃H₂, PO(O-Alkyl)₂ bedeuten, und wobei Alkyl für eine aliphatische Kohlenstoffgruppe mit 1 bis 18 C-Atomen, die linear, verzweigt und/oder auch cyclisch ist, steht und Aryl einen 4 bis zu 14 C-Atome enthaltenden fünf-, sechs- oder siebengliedrigen aromatischen Ring, wobei dieser Ring anelliert sein kann und 0 bis 3 Heteroatome wie N, O, S enthalten kann, bedeutet, wobei sowohl die Alkyl- als auch die Arylgruppe gegebenenfalls bis zu sechs weitere Substituenten tragen, die unabhängig voneinander für Wasserstoff, Alkyl, O-Alkyl, OCO-Alkyl, O-Aryl, Aryl, Fluor, Chlor, Brom, Iod OH, NO₂, NO, SiAlkyl₃, CN, COOH, CHO, SO₃H, NH₂, NH-Alkyl, N-Alkyl₂, PO-Alkyl₂, SO₂-Alkyl, SO-Alkyl, CF₃, NHCO-Alkyl, COO-Alkyl, CONH₂, CO-Alkyl, NHCOH, NHCOO-Alkyl, CO-Aryl, COO-Aryl, PO-Aryl₂, PO₃H₂, PO(O-Alkyl)₂, SO₃-Alkyl bedeuten, wobei Alkyl und Aryl die oben genannten Bedeutungen haben, **gekennzeichnet dadurch, dass** Olefine der allgemeinen Formel (II)
R¹R²C=CR³R⁴ (II)
worin
R¹ bis R⁴ die oben genannten Bedeutungen besitzen,
mit molekularem Sauerstoff in Gegenwart einer Osmiumverbindung, einem chiralen Aminliganden in Wasser oder einem Wasser enthaltenden Lösemittelgemisch bei einem pH-Wert von 8,5 bis 13 umgesetzt werden.

2. Verfahren nach Anspruch 1 zur Herstellung von Verbindungen der Formel (I), **dadurch gekennzeichnet, dass** Olefine der Formel (II) eingesetzt werden, wobei die Substituenten R¹ bis R⁴ unabhängig voneinander Wasserstoff, Alkyl, CN, COOH, COO-Alkyl, COO-Aryl, CO-Alkyl, CO-Aryl, O-Alkyl, O-Aryl, N-Alkyl₂, Aryl, Fluor, Chlor, Brom, Iod, CHO, CF₃, NHCO-Alkyl, CONH₂, CONH-Alkyl, NHCOO-Alkyl bedeuten, und Alkyl und Aryl dabei die oben genannten Bedeutungen besitzen.

3. Verfahren nach Anspruch 1 oder 2, **dadurch gekennzeichnet, dass** chirale Diole der Formel (I) hergestellt werden, worin R¹ bis R⁴ unabhängig voneinander Wasserstoff, Alkyl, CN, COOH, COO-Alkyl, CO-Alkyl, CO-Aryl, O-Alkyl, -Aryl, Aryl, Fluor, Chlor, Brom, CHO, NHCO-Alkyl bedeuten und Alkyl und Aryl die oben genannten Bedeutungen besitzen.

4. Verfahren nach einem der Ansprüche 1 bis 3, **dadurch gekennzeichnet, dass** als Reaktionsmedium eine wässrige Lösung, Olefin und ein organisches Lösemittel verwendet werden.

5. Verfahren nach einem der Ansprüche 1 bis 4, **dadurch gekennzeichnet, dass** als organisches Lösemittel aliphatische Ether, aromatische oder aliphatische Kohlenwasserstoffe, Alkohole und Ester, halogenierte Kohlenwasserstoffe, dipolar aprotische Lösungsmittel wie Dialkylsulfoxide, N,N-Dialkylamide von aliphatischen Carbonsäuren sowie deren Gemische verwendet werden.

6. Verfahren nach einem der Ansprüche 1 bis 5, **dadurch gekennzeichnet, dass** als Oxidationsmittel Sauerstoff oder eine Gasmischung, die mindestens 15 Volumenprozent Sauerstoff enthält, verwendet werden.

7. Verfahren nach einem der Ansprüche 1 bis 6, **dadurch gekennzeichnet, dass** die Reaktion bei Temperaturen von 20 bis 150°C, vorzugsweise von 30 bis 120°C, besonders bevorzugt 40 bis 80°C abläuft, wobei der Druck bis zu 200 bar betragen kann.

8. Verfahren nach einem der Ansprüche 1 bis 7, **dadurch gekennzeichnet, dass** als chirale Amine Diaminocyclohexanderivate, substituierte Diaminoethane, Bis-piperazin, Bis-pyrrolidon-, Bis-tetrahydropyridin-Verbindungen, 1,4-Diazabicyclo[2,2,2]octan-Derivate, substituierte Isooxazolidine, insbesondere (DHQD)₂PHAL (Hydrochinidin-1,4-phthalazindiyl-diether) und (DHQ)₂PHAL (Hydrochinin-1,4-phthalazindiyl-diether) sowie (DHQ)₂Pyr [Hydrochinin-(2,5-diphenyl-4,6-pyrimidinyl)-diether] eingesetzt werden.

9. Verfahren nach einem der Ansprüche 1 bis 8, **dadurch gekennzeichnet, dass** Sulfonamide wie Methylsulfonsäureamid und/oder Carbonsäureamide als CoKatalysatoren zugesetzt werden.

10. Verfahren nach einem der Ansprüche 1 bis 9, **dadurch gekennzeichnet, dass** als Katalysatoren und/oder Präkatalysatoren die Osmiumverbindungen OsO₄, K₂Os₂(OH)₄, Na₂Os₂(OH)₄, Os₃(CO)₁₂, OsCl₃, H₂OsCl₆, [CF₃SO₃Os(NH₃)₅](O₃SCF₃)₂, OsO₄ auf Vinylpyridin, Bu^{t}NOsO₃ eingesetzt werden.

11. Verfahren nach einem der Ansprüche 1 bis 10, **dadurch gekennzeichnet, dass** die eingesetzten Osmiumkatalysatoren zwischen 0,2 und 0,00001 Äquivalente, bezogen auf Olefm, bevorzugt 0,1 bis 0,0001 und besonders bevorzugt 0,08 bis 0,0005 Äquivalente verwendet werden.

12. Verfahren nach einem der Ansprüche 1 bis 11, **dadurch gekennzeichnet, dass** das Verhältnis zu Osmium zwischen 0,01 : 1 bis 1000 : 1, vorzugsweise zwischen 0,1 bis 1 bis 100 : 1, besonders bevorzugt von 1 : 1 bis 50 : 1 beträgt.

## Claims

1. Process for the asymmetric dihydroxylation of olefins by means of osmium catalysts to prepare monofunctional, bifunctional and/or polyfunctional chiral 1,2-diols of the formula (I)
R¹R²C(OH)-C(OH)R³R⁴ (I)
where
R¹ to R⁴ are each, independently of one another, hydrogen, alkyl, CN, COOH, COO-alkyl, COO-aryl, CO-alkyl, CO-aryl, O-alkyl, O-aryl, O-CO-aryl, O-CO-alkyl, OCOO-alkyl, N-alkyl₂, NH-alkyl, N-aryl₂, NH-aryl, NO, NO₂, NOH, aryl, fluorine, chlorine, bromine, iodine, NO₂, Si-alkyl₃, CHO, SO₃H, SO₃-alkyl, SO₂-alkyl, SO-alkyl, CF₃, NHCO-alkyl, CONH₂, CONH-alkyl, NHCOH, NHCOO-alkyl, CHCHCO₂-alkyl, CHCHCO₂H, PO-(aryl)₂, PO-(alkyl)₂, PO₃H₂, PO(O-alkyl)₂, where alkyl represents an aliphatic organic group having from 1 to 18 carbon atoms which may be linear, branched and/or cyclic and aryl is a five-, six- or seven-membered aromatic ring which contains from 4 to 14 carbon atoms and may be fused and contain from 0 to 3 hetero atoms such as N, 0, S and where the alkyl and/or the aryl group may bear up to six further substituents selected independently from among hydrogen, alkyl, O-alkyl, OCO-alkyl, 0-aryl, aryl, fluorine, chlorine, bromine, iodine, OH, NO₂, NO, Si-alkyl₃, CN, COOH, CHO, SO₃H, NH₂, NH-alkyl, N-alkyl₂, PO-alkyl₂, SO₂-alkyl, SO-alkyl, CF₃, NHCO-alkyl, COO-alkyl, CONH₂, CO-alkyl, NHCOH, NHCOO-alkyl, CO-aryl, COO-aryl, PO-aryl₂, PO₃H₂, PO(O-alkyl)₂, SO₃-alkyl, where alkyl and aryl are as defined above, **characterized in that** olefins of the formula (II)
R¹R²C=CR³R⁴ (II)
where
R¹ to R⁴ are as defined above,
are reacted with molecular oxygen in the presence of an osmium compound, a chiral amine ligand in water or a water-containing solvent mixture at a pH of from 8.5 to 13.

2. Process according to Claim 1 for preparing compounds of the formula (I), **characterized in that** olefins of the formula (II) in which the substituents R¹ to R⁴ are each, independently of one another, hydrogen, alkyl, CN, COOH, COO-alkyl, COO-aryl, CO-alkyl, CO-aryl, 0-alkyl, O-aryl, N-alkyl₂, aryl, fluorine, chlorine, bromine, iodine, CHO, CF₃, NHCO-alkyl, CONH₂, CONH-alkyl, NHCOO-alkyl and alkyl and aryl are as defined above are used.

3. Process according to Claim 1 or 2, **characterized in that** chiral diols of the formula (I) in which R¹ to R⁴ are each, independently of one another, hydrogen, alkyl, CN, COOH, COO-alkyl, CO-alkyl, CO-aryl, 0-alkyl, O-aryl, aryl, fluorine, chlorine, bromine, CHO, NHCO-alkyl and alkyl and aryl are as defined above are prepared.

4. Process according to any of Claims 1 to 3, **characterized in that** the reaction medium used comprises an aqueous solution, olefin and an organic solvent.

5. Process according to any of Claims 1 to 4, **characterized in that** aliphatic ethers, aromatic or aliphatic hydrocarbons, alcohols and esters, halogenated hydrocarbons, dipolar aprotic solvents such as dialkyl sulphoxides, N,N-dialkylamides of aliphatic carboxylic acids and mixtures thereof are used as organic solvent.

6. Process according to any of Claims 1 to 5, **characterized in that** the oxidant used is oxygen or a gas mixture comprising at least 15% by volume of oxygen.

7. Process according to any of Claims 1 to 6, **characterized in that** the reaction proceeds at temperatures of from 20 to 150°C, preferably from 30 to 120°C, particularly preferably from 40 to 80°C, and a pressure of up to 200 bar.

8. Process according to any of Claims 1 to 7, **characterized in that** the chiral amines used are diaminocyclohexane derivatives, substituted diaminoethanes, bispiperazine, bispyrrolidone and bistetrahydropyridine compounds, 1,4-diazabicyclo[2.2.2]octane derivatives, substituted isooxazolidines, in particular (DHQD)₂PHAL (hydroquinidine 1,4-phthalazinediyl diether) and (DHQ)₂PHAL (hydroquinine 1,4-phthalazinediyl diether) and (DHQ)₂Pyr (hydroquinine 2,5-diphenyl-4,6-pyrimidinyl diether).

9. Process according to any of Claims 1 to 8, **characterized in that** sulphonamides such as methylsulphonamide and/or carboxamides are added as cocatalysts.

10. Process according to any of Claims 1 to 9, **characterized in that** the osmium compounds OsO₄, K₂Os₂(OH)₄, Na₂Os₂(OH)₄, Os₃(CO)₁₂, OsCl₃, H₂OsCl₆, [CF₃SO₃Os(NH₃)₅] (O₃SCF₃)₂, OsO₄ on vinylpyridine, Bu^{t}NOsO₃ are used as catalysts and/or catalyst precursors.

11. Process according to any of Claims 1 to 10, **characterized in that** the osmium catalysts employed are used in amounts of from 0.2 to 0.00001 equivalents, based on olefin, preferably from 0.1 to 0.0001 equivalents and particularly preferably from 0.08 to 0.0005 equivalents.

12. Process according to any of Claims 1 to 11, **characterized in that** the ratio of amine to osmium is from 0.01:1 to 1 000:1, preferably from 0.1:1 to 100:1, particularly preferably from 1:1 to 50:1.

## Revendications

1. Procédé pour la dihydroxylation asymétrique d'oléfines à l'aide de catalyseurs à base d'osmium en vue de préparer des 1,2-diols mono-, bi- et/ou poly-fonctionnels chiraux de formule (I)
R¹R²C(OH)-C(OH)R³R⁴ (I)
dans laquelle
R¹ à R⁴ représentent chacun, indépendamment les uns des autres l'hydrogène, un groupe alkyle, CN, COOH, COO-alkyle, COO-aryle, CO-alkyle, CO-aryle, O-alkyle, O-aryle, O-CO-aryle, O-CO-alkyle, OCOO-alkyle, N-alkyle₂, NH-alkyle, N-aryle₂, NH-aryle, NO, NO₂, NOH, aryle, le fluor, le chlore, le brome, l'iode, un groupe NO₂, Sialkyle₃, CHO, SO₃H, SO₃-alkyle, SO₂-alkyle, SO-alkyle, CF₃, NHCO-alkyle, CONH₂, CONH-alkyle, NHCOH, NHCOO-alkyle, CHCHCO₂-alkyle, CHCHCO₂H, PO-(aryle)₂, PO-(alkyle)₂, PO₃H₂, PO(O-alkyle)₂, les groupes alkyle en question étant des groupes hydrocarbonés aliphatiques en C₁ à C₁₈, linéaires, ramifiés et/ou cycliques, et les groupes aryle en question des cycles aromatiques à 5, 6 ou 7 chaînons contenant 4 à 14 atomes de carbone, ces cycles pouvant être condensés et pouvant contenir 0 à 3 hétéroatomes tels que N, O, S, ces groupes alkyle et aryle pouvant le cas échéant porter eux-mêmes jusqu'à 6 autres substituants choisis, indépendamment les uns des autres, parmi l'oxygène, les groupes alkyle, O-alkyle, OCO-alkyle, O-aryle, aryle, le fluor, le chlore, le brome, l'iode, les groupes OH, NO₂, NO, Sialkyle₃, CN, COOH, CHO, SO₃H, NH₂, NH-alkyle, N-alkyle₂, PO-alkyle₂, SO₂-alkyle, SO-alkyle, CF₃, NHCO-alkyle, COO-alkyle, CONH₂, CO-alkyle, NHCOH, NHCOO-alkyle, CO-aryle, COO-aryle, PO-aryle₂, PO₃H₂, PO(O-alkyle)₂, SO₃-alkyle, ces derniers groupes alkyle et aryle étant les mêmes que ci-dessus, **caractérisé en ce que** l'on fait réagir des oléfines de formule générale (II)
R¹R²C=CR³R⁴ (II)
dans laquelle
R¹ à R⁴ ont les significations indiquées ci-dessus,
avec l'oxygène moléculaire en présence d'un dérivé de l'osmium, d'un ligand chiral du type amine dans l'eau ou dans un mélange solvant contenant de l'eau à un pH de 8,5 à 13.

2. Procédé selon la revendication 1 pour la préparation des composés de formule (I), **caractérisé en ce que** l'on part d'oléfines de formule (II) dans laquelle les symboles R¹ à R⁴ représentent chacun, indépendamment les uns des autres, l'hydrogène, un groupe alkyle, CN, COOH, COO-alkyle, COO-aryle, CO-alkyle, CO-aryle, O-alkyle, O-aryle, N-alkyle₂, aryle, le fluor, le chlore, le brome, l'iode, un groupe CHO, CF₃, NHCO-alkyle, CONH₂, CONH-alkyle, NHCOO-alkyle, les groupes alkyle et aryle en question étant les mêmes que ceux qui ont été cités ci-dessus.

3. Procédé selon la revendication 1 ou 2, **caractérisé en ce que** l'on prépare des diols chiraux de formule (I) dans laquelle R¹ à R⁴ représentent chacun, indépendamment les uns des autres, l'hydrogène, un groupe alkyle, CN, COOH, COO-alkyle, CO-alkyle, CO-aryle, O-alkyle, O-aryle, aryle, le fluor, le chlore, le brome, un groupe CHO, NHCO-alkyle, les groupes alkyle et aryle en question étant les mêmes que ci-dessus.

4. Procédé selon l'une des revendications 1 à 3, **caractérisé en ce que** le milieu de réaction consiste en une solution aqueuse, l'oléfine et un solvant organique.

5. Procédé selon l'une des revendications 1 à 4, **caractérisé en ce que** le solvant organique utilisé consiste en un éther aliphatique, un hydrocarbure aromatique ou aliphatique, un alcool ou un ester, un hydrocarbure halogéné, un solvant aprotonique bipolaire tel qu'un dialkylsulfoxyde, un N,N-dialkylamide d'acide carboxylique aliphatique ou leurs mélanges.

6. Procédé selon l'une des revendications 1 à 5, **caractérisé en ce que** l'agent oxydant utilisé est l'oxygène ou un mélange gazeux contenant au moins 15 % en volume d'oxygène.

7. Procédé selon l'une des revendications 1 à 6, **caractérisé en ce que** la réaction est réalisée à des températures allant de 20 à 150°C, de préférence de 30 à 120°C, plus spécialement de 40 à 80°C sous une pression qui peut aller jusqu'à 200 bar.

8. Procédé selon l'une des revendications 1 à 7, **caractérisé en ce que** les amines chirales utilisées sont des dérivés du diaminocyclohexane, des diaminoéthanes substitués, la bis-pipérazine, des dérivés de bis-pyrrolidone ou de bis-tétrahydropyridine, des dérivés du 1,4-diazabicyclo[2,2,2]octane, des isooxazolidines substituées, en particulier le (DHQD)₂PHAL (diéther 1,4-phtalazinediylique de l'hydroquinidine) et le (DHQ)₂PHAL (diéther 1,4-phtalazinediylique de l'hydroquinine) ou le (DHQ)₂Pyr (diéther 2,5-diphényl-4,6-pyrimidinylique de l'hydroquine).

9. Procédé selon l'une des revendications 1 à 8, **caractérisé en ce que** l'on ajoute, en tant que catalyseurs auxiliaires, des sulfonamides comme le méthylsulfonamide et/ou des carboxamides.

10. Procédé selon l'une des revendications 1 à 9, **caractérisé en ce que** l'on utilise en tant que catalyseurs et/ou pré-catalyseurs les dérivés de l'osmium OsO₄, K₂Os₂(OH)₄, Na₂Os₂(OH)₄, Os₃(CO)₁₂, OsCl₃, H₂OsCl₆, [CF₃SO₃Os(NH₃)₅](O₃SCF₃)₂, OsO₄ sur vinylpyridine, Bu^{t}NOsO₃.

11. Procédé selon l'une des revendications 1 à 10, **caractérisé en ce que** les catalyseurs à base d'osmium sont utilisés en quantité de 0,2 à 0,00001 équivalent par rapport à l'oléfine, de préférence en quantité de 0,1 à 0,0001 et plus spécialement de 0,08 à 0,0005 équivalent.

12. Procédé selon l'une des revendications 1 à 11, **caractérisé en ce que** le rapport à l'osmium va de 0,01:1 à 1000:1, de préférence de 0,1 à 100:1 et plus spécialement de 1:1 à 50:1.
